(19) Europäisches Patentamt
European Patent Office
Office européen des brevets

(11) **EP 0 801 561 B1**

(12) **EUROPEAN PATENT SPECIFICATION**

(45) Date of publication and mention
of the grant of the patent:
**06.04.2005 Bulletin 2005/14**

(51) Int Cl.[7]: **A61K 9/70**, A61K 47/10,
A61K 47/18

(21) Application number: **96902047.8**

(22) Date of filing: **11.01.1996**

(86) International application number:
**PCT/US1996/000015**

(87) International publication number:
**WO 1996/020699 (11.07.1996 Gazette 1996/31)**

(54) **COMPOSITION AND METHODS FOR TRANSDERMAL DELIVERY OF ACID-LABILE DRUGS**

MITTEL UND VERFAHREN ZUR TRANSDERMALEN VERABREICHUNG SÄURELABILER
ARZNEISTOFFE

COMPOSITION ET PROCEDES POUR L'ADMINISTRATION TRANSDERMIQUE DE
MEDICAMENTS LABILES SOUS L'EFFET D'UN ACIDE

(84) Designated Contracting States:
**AT BE CH DE DK ES FR GB GR IE IT LI LU MC NL
PT SE**
Designated Extension States:
**LT LV SI**

(30) Priority: **06.01.1995 US 369756**

(43) Date of publication of application:
**22.10.1997 Bulletin 1997/43**

(73) Proprietor: **NOVEN PHARMACEUTICALS, INC.**
**Miami, FL 33186 (US)**

(72) Inventor: **LI, Chensheng**
**Miami, FL 33186 (US)**

(74) Representative: **Lee, Nicholas John et al**
**Kilburn & Strode,**
**20 Red Lion Street**
**London WC1R 4PJ (GB)**

(56) References cited:
**EP-A- 0 196 769       EP-A- 0 275 716**
**EP-A- 0 379 045       EP-A- 0 483 105**
**EP-A- 0 573 133       WO-A-92/10154**
**WO-A-93/08795       WO-A-95/18603**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

**Description**

The present invention relates generally to compositions and devices for the transdermal delivery of acid-labile drugs. The present invention relates more particularly to a drug penetration-enhancing transdermal composition, wherein the penetration enhancer is a functional derivative of a fatty acid that the functional derivative minimizes the acid catalyzed degradation of acid-labile drugs.

Transdermal drug delivery systems are effective means for introducing drugs into the bloodstream by applying them to skin or mucosa. Advantages of transdermal delivery include convenience, comfort, avoidance of the risks associated with enteral and parenteral administration, and control over drug absorption. It is particularly suitable for treating conditions that are systemic in nature, and can be used with any drug that can pass through the skin or mucosa and that do not irritate, or cause an allergic reaction, at the site of application. Permeability of a drug through the skin may be affected by the stratum corneum, which is characterized by dehydrated and keratinized cells, or by the epidermal layer in the mucosa. The art has recognized that the barriers to the transdermal or percutaneous delivery of drug through the skin can be overcome or reduced by using particular vehicles and carriers into which the drug is incorporated, so that the vehicle or carrier remains at the site of application and increases the drug's penetration at that site.

U.S. Patent No. 4,863,970 by Patel (1989) describes compositions and methods for improving the penetration of pharmaceutically-active agents that are lipophilic or hydrophilic, including salts thereof. The topical compositions of Patel contain a pharmaceutically-active agent dissolved in, or admixed with, a penetration-enhancing binary mixture of (a) one or more cell-envelope disordering compounds such as oleic acid, and (b) a lower alkanol (e.g., C2 or C3 lower alcohol). In addition, the formulation can optionally contain inert hydrophilic or hydrophobic ingredients which are soluble with the enhancer composition. This patent does not recognize the deleterious effects on acid-labile drugs of a penetration enhancer such as oleic acid, nor does it suggest the property of functional derivatives of fatty acids to stabilize drugs subject to acid catalyzed degradation, and at the same time function as penetration enhancers.

U.S. Patent No. 5,162,315 describes the use of certain substituted alkanamides as penetration enhancers, but does not suggest their particular suitability in stabilizing acid-labile drugs.

Cooper, E., "Increased Skin Permeability for Lipophilic Molecules" *J. Pharm. Sci.* 73: (Aug. 1984) describes the use of oleic acid in different concentrations in the presence of propylene glycol or 1,1-butanediol, but does not recognize the deleterious effect of the penetration enhancer on acid-labile drugs.

European Patent Application No. 573 133 discloses a composition for the transdermal delivery of gestodene, optionally in combination with one or more estrogens. This composition also contains a volatile solvent and a penetration enhancer. Solvents include a lower alcohol, ketone, or lower carboxylic acid ester, such as ethanol, isopropanol, acetone, or ethyl acetate, a polar ether, such as tetrahydrofuran, lower hydrocarbon, such as cyclohexane or naphtha, or also a halogenated hydrocarbon, such as dichloromethane, trichloromethane, trichlorotrifluoroethane, and trichlorofluoromethane, or mixtures of the above. Penetration enhancers include alcohols, such as 1,2-propanediol or benzyl alcohol, saturated and unsaturated fatty alcohols with 8 to 18 carbon atoms, such as lauryl alcohol or cetyl alcohol, hydrocarbons, such as mineral oil, saturated and unsaturated fatty acids of 8 to 18 carbon atoms, such as stearic acid or oleic acid, and fatty acid esters. Fatty acid esters include lauric acid, myristic acid, stearic acid and palmitic acid.

European Patent Application No. 196 769 discloses a transdermal pharmaceutical polymer which contains a skin absorption enhancer. The enhancer may be oleic acid, propyl oleate, decyl methyl sulfoxide and 1-dodecylazacycloheptan-2-one.

European Patent Application No. 0573133 discloses a composition for the transdermal administration of gestoden.

Although penetration enhancers are well-known in the art, problems associated with combining certain penetration enhancers with certain drugs in transdermal compositions have not been addressed. For instance, the applicant has observed that oleic acid, which is a commonly used penetration enhancer, will react with certain drugs, not only causing them to degrade but also creating by-products that interfere with drug penetration and delivery. The applicant has discovered that, in order to overcome this type of problem, functional derivatives of fatty acids can be used in transdermal formulations containing drugs that degrade in the presence of weak organic acids such as oleic acid, particularly over prolonged periods of time, for example, for weeks or months during storage. Such drugs are referred to herein as "acid-labile" drugs and include esters, α,β-unsaturated ketones, aminolevulinic acids, amides, imines and similar compounds subject to degradation in the presence of weak organic acids. The applicant was the first to appreciate the above described problem and solution.

The term "fatty acid" is used here in the broadest sense to include saturated and unsaturated, straight and branched chain aliphatic acids having from four to twenty-four carbon atoms.

The term "functional derivative" is used here to refer to isosteric modifications of fatty acids or to non-acidic derivatives of the carboxylic acid functional group of a fatty acid or isosteric modifications thereof.

## Summary of the Invention

In a first aspect, the present invention provides a transdermal drug penetration enhancing composition comprising (a) a therapeutically effective amount of a pharmacologically active, acid-labile drug that is subject to acid catalysed degradation; (b) a pharmaceutically acceptable carrier substantially free of a lower alkanol; and (c) a penetration-enhancing amount of a functional derivative of a fatty acid, wherein said derivative is selected from the group consisting of amides, alcohols, and polyols.

In a second aspect, the present invention provides the use of: a) a therapeutically effective amount of a pharmacologically active, acid-labile drug which is subject to acid catalysed degradation; (b) a pharmaceutically acceptable carrier substantially free of a lower alkanol; and (c) a penetration-enhancing amount of a functional derivative of a fatty acid, wherein said derivative is selected from the group consisting of amides, alcohols, and polyols, in the manufacture of a topical medicament for enhancing the penetration of a pharmaceutically active compound.

In a third aspect, the present invention provides a stabilised adhesive type transdermal device for delivery of a compound, said compound being stable upon extended storage of said device, comprising an effective amount of said compound in a carrier which is substantially free of lower alkanols, and a penetration-enhancing amount of a functional derivative of a fatty acid, wherein said derivative is selected from the group consisting of amides, alcohols and polyols and wherein said compound is selected from the group consisting of progesterone, ethisterone, medroxyprogesterone, $17\alpha$-hydroxyprogesterone, norethindrone, norethindrone acetate, dydrogesterone, chlomadinone acetate, norgestrel and esters.

## Brief Description of the Drawings

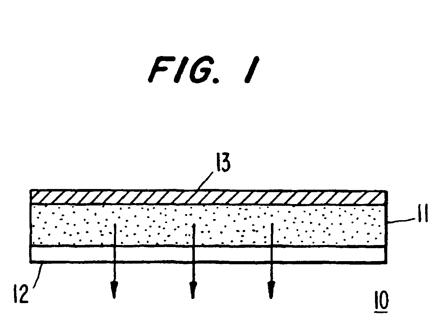

FIGURE 1 shows a schematic illustration of an adhesive monolithic device, in accordance with the present invention.

## Detailed Description of the Preferred Embodiments

By "substantially free of a lower alkanol" is meant less than 5%, and preferably less than 2%, of lower alkanols. For the preferred adhesive carrier, lower alkanols and processing solvents are driver off during processing.

As discussed above, the term "functional derivative of a fatty acid" means isosteric modifications of fatty acids or non-acidic derivatives of the carboxylic functional group of a fatty acid or isosteric modifications thereof. Specifically, a functional derivative of a fatty acid includes a saturated, or preferably unsaturated (all cis), aliphatic acid in which the carboxyl group is substituted with a functional derivative thereof, such as alcohols, polyols, amides, and substituted derivatives thereof, including esters, ethers and N,N' disubstituted amides. In the present invention, the derivative is selected from the group consisting of alcohols, polyols and amides. The term "fatty acid" as used here and as noted above means higher molecular weight, straight and branched chain saturated and unsaturated aliphatic acids of four to twenty-four carbons, including but not limited to those having an even number of carbon atoms, derived from animals and plants. The expression "functional derivative of a fatty acid" is intended to include at least one functional derivative of a fatty acid.

As used herein, the term "pharmacologically active drug" and its equivalents, e.g., "agent," "bioactive agent," and "medicament," are intended to have the broadest meaning and includes at least one of any therapeutically, prophylactically and/or pharmacologically or physiologically beneficial active substance, or mixture thereof, which is delivered to a living organism to produce a desired, usually beneficial, effect.

More specifically, any drug which is capable of producing a pharmacological response, localized or systemic, irrespective of whether therapeutic, diagnostic, or prophylactic in nature, particularly in animals, is within the contemplation of the invention. It should be noted that the drugs and/or bioactive agents may be used singularly or as a mixture of two or more such agents, and in amounts sufficient to prevent, cure, diagnose or treat a disease or other condition, as the case may be.

However, the drugs for use in this invention are those which are acid-labile, namely those that are degraded by acids, even organic acids, or are degraded in acid catalyzed reactions. The invention is, therefore, particularly suitable for use with $\alpha$, $\beta$-unsaturated ketones, especially 3-oxo-4, 5 dehydrosteroids, esters, and $\alpha$-ketoamino acids and imines. The acid-labile drug of the present invention may be norethindrone acetate. Other acid-labile drugs which may be included are:

**ANABOLIC STEROIDS** such as Androisoxazole, Bolasterone, Clostebol, Ethylestrenol, Formyldienolone, 4-Hydroxy-19-nortestosterone, Methenolone, Methyltrienolone, Nandrolone, Nandrolone Decanoate, Nandrolone p-Hexyloxyphenyl-propionate, Nandrolone Phenpropionate, Norbolethone, Oxymesterone, Quinbolone, Stenbolone, Trenbolone

**ANDROGENIC STEROIDS** such as Boldenone, Fluoxymesterone, Mestanolone, Mesterolone, Methandrostenolone, 17-Methyltestosterone, 17$\alpha$-Methyl-testosterone 3-Cyclopentyl Enol Ether, Norethandrolone, Normethandrone, Oxandrolone, Oxymetholone, Prasterone, Stanlolone, Stanozolol, Testosterone, Testosterone 17-Chloral Hemiacetal, Testosterone 17$\beta$-Cypionate, Testosterone Enanthate, Testosterone Nicotinate, Testosterone Phenylacetate, Testosterone Propionate, Tiomesterone

**DIURETIC STEROIDS** such as Canrenone, Oleandrin, Spironolactone, and

**PROGESTOGENS** such as Anagestone, Chlormadinone Acetate, Delmadinone Acetate, Demegestone, Dimethisterone, Dydrogesterone, Ethinylestrenol, Ethisterone, Ethynodiol, Ethynodiol Diacetate, Flurogestone Acetate, Gestodene, Gestonorone Caproate, Haloprogesterone, 17-Hydroxy-16-methylene--progesterone, 17$\alpha$-Hydroxyprogesterone, 17$\alpha$-Hydroxyprogesterone Caproate, Medrogestone, Medroxyprogesterone, Megestrol Acetate, Melengestrol, Norethindrone, Norethindrone Acetate, Norethynodrel, Norgesterone, Norgestimate, Norgestrel, Norgestrienone, 19-Norprogesterone, Norvinisterone, Pentagestrone, Progesterone, Promegestone, Quingestrone, Trengestone

The above list of pharmaceutical agents is based upon the list provided in *The Merck Index,* 11th Edition, Merck & Co. Rahway, N.J. (1989).

The drugs and mixtures thereof can be present in the composition of the present invention in different forms, depending on which form yields the optimum delivery characteristics. Thus, the drug, containing acidic or basic groups, respectively, can be in free acid or base form, or in the form of salts, esters, or any other pharmacologically acceptable functional derivative, including components of molecular complexes.

The amount of drug to be incorporated in the composition varies depending on the particular drug, the desired therapeutic effect, and the time span for which the device is to provide therapy. The drug is used in a "pharmacologically effective amount." This means that the concentration of the drug is such that in the composition it results in a therapeutic level of drug delivered over the term that the transdermal dosage form is to be used, preferably with zero order kinetics. Such delivery is dependent on many variables including the chemical nature of the drug, the form of the drug, the time period for which the individual dosage unit is to be used, the flux rate of the drug from the system, and a number of other variables. The amount of drug needed can be experimentally determined based on the flux rate of the drug through the system and through the skin when used with and without enhancers. Having determined the flux rate needed, the transdermal delivery system is designed so that the release rate over the period of time of therapeutic use will be at least equal to the flux rate. Of course, the surface area of the transdermal delivery system also affects the delivery of the drug from the system.

In general, therapeutic amounts of drug can be delivered from the composition containing 0.05% to about 50% by weight of drug, or more preferably from about 0.1% to about 30% by weight. However, the composition of this invention is particularly useful for drugs that are used in relatively low concentrations, especially 0.1% to 20% of the total composition.

The carrier of the present invention can be at least one of any pharmaceutically acceptable carrier which is capable of conforming to a surface with which it comes into contact and capable of maintaining the contact so as to facilitate topical application without any adverse physiological response, and which maintain their form and do not appreciably decompose in use. Such carriers can be, among other things, creams, ointments, oils, lubricants and adhesives.

In the preferred embodiment, the carrier is an adhesive or combinations of adhesives such as any of the nontoxic polymers, particularly those known in the art to carry drugs for transdermal delivery, including natural or synthetic elastomers, such as polyisobutylene, styrene, butadiene, styrene isoprene block copolymers, acrylics, urethanes, silicones, styrene butadiene copolymers, acrylic acid polymers, polyacrylates, and polysaccharides 'such as karaya gum, tragacanth gum, pectin, guar gum, cellulose, and cellulose derivative such as methyl cellulose, propyl cellulose, cellulose acetate and the like, along with other substances capable of forming a solid colloid that can adhere to the skin or mucosa, alone or in combination with other carriers. The term "adhesive" means a substance, inorganic or organic, natural or synthetic that is capable of surface attachment at the intended application site.

In the most preferred embodiment, the carrier of the present invention is a pressure sensitive adhesive, *i.e.* a viscoelastic material which adheres instantaneously to most substrates with the application of very slight pressure and remains permanently tacky. Preferably, the pressure sensitive adhesive comprises a blend of at least two polymers and a soluble polyvinylpyrrolidone ("PVP").

The soluble PVP is preferably used in an amount effective to solubilize the drug.

A polymer is a pressure-sensitive adhesive within the meaning of the term as used herein if it has the properties of a pressure-sensitive adhesive *per se* or functions as a pressure-sensitive adhesive by admixture with tackifiers, plasticizers or other additives. The term pressure-sensitive adhesive also includes mixtures of different polymers and mixtures of polymers, such as polyisobutylenes (PIB) of different molecular weights, the resultant mixtures being a pressure-sensitive adhesive. In the last case, the polymers of lower molecular weight in the mixture are not considered to be "tackifiers," said term being reserved for additives which differ other than in molecular weight from the polymers to which they are added.

As used herein, the term "rubber" refers to a viscoelastic material which has the properties of a pressure-sensitive adhesive and which contains at least one natural or synthetic elastomeric polymer. Suitable rubbers include polysiloxane, polyisobutylene and natural rubber.

The multiple polymer adhesive system is preferably formulated so that it is a pressure-sensitive adhesive at room temperature and has other desirable characteristics for adhesives used in the transdermal drug delivery art. Such characteristics include good adherence to skin, ability to be peeled or otherwise removed without substantial trauma to the skin, retention of tack with aging, etc. In general, the multiple polymer adhesive system should have a glass transition temperature (Tg), measured using a differential scanning calorimeter, of between about -70°C and 0°C.

The term "acrylic polymer" is used herein as in the art interchangeably with polyacrylate, polyacrylic and acrylic adhesive. The acrylic-based polymer and. silicone-based polymer are preferably in a ratio by weight, respectively, from about 2:98 to about 96:4, more preferably from about 2:98 to about 90:10, and even more preferably about 2:98 to about 86:14.

Suitable acrylic adhesives are commercially available and include the polyacrylate adhesives sold under the trademarks Duro-Tak 80-1194, 80-1196, 80-1197, 2287, 2516 and 2852 by National Starch and Chemical Corporation, Bridgewater, New Jersey. Other suitable acrylic adhesives are those sold under the trademarks Gelva-Multipolymer Solution GMS 737, 788, 1151 and 1430 (Monsanto; St. Louis, MO).

The rubber adhesives useful in practicing the invention include hydrocarbon polymers such as natural and synthetic polyisoprene, polybutylene and polyisobutylene, styrene/butadiene polymers, styrene-isoprene-styrene block copolymers, hydrocarbon polymers such as butyl rubber, halogen-containing polymers such as polyacrylo-nitrile, polytetrafluoroethylene, polyvinylchloride, polyvinylidene chloride, and polychloropene, and polysiloxanes and other copolymers thereof.

Suitable polysiloxanes include silicone pressure-sensitive adhesives which are based on two major components: a polymer, or elastomer, and a tackifying resin. The polysiloxane adhesive is usually prepared by cross-linking the elastomer, typically a high molecular weight polydiorganosiloxane, with the resin, to produce a three-dimensional siloxane structure, via a condensation reaction in an appropriate organic solvent. The ratio of resin to elastomer is the most important factor which can be adjusted in order to modify the physical properties of polysiloxane adhesives. Sobieski, *et al.*, "Silicone Pressure Sensitive Adhesives, " <u>Handbook of</u> <u>Pressure-Sensitive Adhesive Technology, 2nd ed.</u>, pp. 508-517 (D. Satas, ed.), Van Nostrand Reinhold, New York (1989).

Suitable silicone pressure-sensitive adhesives are commercially available and include the silicone adhesives sold under the trademarks BIO-PSA(as described in U.S. Patent No. 4,655,767), X7-3027(methylated trimethylated silica in heptane), X7-4203(methylated trimethylated silica in toluene), Q7-4503(trimethylated silica treated with dimethyl siloxane, in tolune, as described at Chemical Abstract Registry No. 068440700), X7-4603 (trimethylated silica treated with dimethyl siloxane, in toluene, as described at Chemical Abstract Registry No. 068440700), X7-4301(methylated trimethylated silica in heptane), X7-4303(methylated trimethylated silica in toluene), X7-4919(trimethylated silica treated with dimethyl siloxane, in heptane as describe at Chemical Abstract Registry No. 068440700), X7-2685(trimethylated silica treated with dimethyl siloxane, in toluene, as described at Chemical Abstract Registry No. 068440700), and X7-3122(trimethylated silica treated with dimethyl siloxane, in heptane, as described at Chemical Abstract Registry No. 068440700) by Dow Corning Corporation, Medical Products, Midland, Michigan. BIO-PSA X7-4203, X7-4301 and X7-4303 are particularly suitable for use in formulations containing amine-functional drugs, such as albuterol.

In the practice of preferred embodiments of the invention, the polysiloxane constitutes preferably from about 9% to about 97% of the total weight of the pressure-sensitive adhesive composition, more preferably about 12% to about 97%, and optimally about 14% to about 94%.

The composition of the present invention may also include a drug solubilizing amount of a solvent. A "drug solubilizing effective amount of a solvent" refers to non-toxic, pharmaceutically acceptable substances, preferably liquids which do not substantially negatively affect the adhesion properties of the system and in which the drugs in the amounts employed are fully soluble. For instance, the solvent may be primarily a polyhydric alcohol or combination of polyhydric alcohols, particularly if the carrier is a gum. The term polyhydric alcohol means any organic polyol. Polyhydric alcohols include glycols, triols and polyols having 4 to 6 alcoholic hydroxyl groups.

In a preferred embodiment, the carrier is a pressure sensitive adhesive comprising a blend of polymers including PVP. Indeed, soluble PVP has been found to be highly effective in solubilization of drugs in adhesive-type transdermal drug delivery systems according to the invention, wherein the carrier is a blend of polymers. In particular, soluble PVP has proved useful in maintaining a norethindrone acetate (NETA) system and an NETA/estradiol system substantially crystal-free. Other specific drugs for which soluble PVP is particularly usefully employed according to the invention include albuterol, estradiol, haloperidol and alprazolam.

The amount and type of soluble PVP required in the foregoing preferred embodiment will depend on the quantity and type of drug present in the adhesive, as well as the type of adhesive.

However, for drug molecules which are not readily soluble in the polymer system containing PVP, another solvent or "co-solvent" for the drug and polymer can be added. Co-solvents, such as lecithin, retinol derivatives, tocopherol, dipropylene glycol, triacetin, propylene glycol, saturated and unsaturated fatty acids, mineral oil, silicone fluid, alcohols, butyl benzyl phthalate, and the like are useful in the practice of the instant invention depending on the solubility of the drug in the multiple polymer adhesive system.

In a preferred embodiment of the invention, a transdermal drug delivery system is prepared by mixing a soluble PVP, polyacrylate, polysiloxane, drug, co-solvent(s), and tackifying agent, if needed, in an appropriate volatile solvent(s), then casting the mixture and removing the solvent(s) by evaporation to form a film.

Suitable volatile solvents include, but are not limited to, alcohols such as isopropanol and ethanol; aromatics such as xylenes and toluene; aliphatics such as hexane, cyclohexane, and heptane; and alkanoic acid esters such as ethyl acetate and butyl acetate.

A "penetration enhancer" is an agent known to accelerate the delivery the drug through the skin. These agents also have been referred to as accelerants, adjuvants, and sorption promoters, and are collectively referred to herein as "enhancers." This class of agents includes those with diverse mechanisms of action including those which have the function of improving the solubility and diffusibility of the drug within the multiple polymer and those which improve percutaneous absorption, for example, by changing the ability of the stratum corneum to retain moisture, softening the skin, improving the skin's permeability, acting as penetration assistants or hair-follicle openers or changing the state of the skin including the boundary layer.

According to the present invention, a "penetration enhancing amount" is an amount up to about 20% by weight of the whole composition, but preferably an amount of about 1% to about 10% by weight.

The penetration enhancer of the present invention is, as discussed above, a functional derivative of a fatty acid, which includes isosteric modifications of fatty acids or non-acidic derivatives of the carboxylic functional group of a fatty acid or isosteric modifications thereof. The functional derivative of a fatty acid is an unsaturated (all *cis*) alkanoic acid in which the carboxyl group is substituted with a functional derivative thereof, such as alcohols, polyols, and amides.

The term "fatty acid" means a fatty acid that has four to twenty-four carbon atoms, and includes but is not limited to those derived from animals and plants. Particular preferred derivatives are those based on oleic acid, e.g., oleyl alcohol, and oleamide as follows:

| Group | Formulation | CTFA Name |
|---|---|---|
| ALCOHOLS | $CH_3(CH_2)_7CH=CH(CH_2)_8OH$ | Oleyl Alcohol |
| | $CH_3(CH_2)_7CH=CH(CH_2)_7CH_2(OCH_2CH_2)_2OH$ | Oleth-2 |
| | $CH_3(CH_2)_7CH=CH(CH_2)_7CH_2(OCH_2CH_2)_5OH$ | Oleth-5 |
| | $CH_3(CH_2)_7CH=CH(CH_2)_7CH_2(OCH_2CH_2)_{10}OH$ | Oleth-10 |
| | $CH_3(CH_2)_7CH=CH(CH_2)_7CH_2(OCH_2CH_2)_{20}OH$ | Oleth-20 |
| | $CH_3(CH_2)_7CH=CH(CH_2)_7CH_2(OCH_2CH_2)_{30}OH$ | Oleth-30 |
| | | |
| | $CH_3(CH_2)_7CH=CH(CH_2)_7CH_2(OCH-CH_2)_{10}OH$<br>$\qquad\qquad\qquad\qquad\quad \underset{CH_3}{\vert}$ | PPG-10<br>Oleyl Ether |
| | $CH_3(CH_2)_7CH=CH(CH_2)_7CH_2(OCH-CH_2)_{20}OH$<br>$\qquad\qquad\qquad\qquad\quad \underset{CH_3}{\vert}$ | PPG-20<br>Oleyl Ether |
| | $CH_3(CH_2)_7CH=CH(CH_2)_7CH_2(OCH-CH_2)_{50}OH$<br>$\qquad\qquad\qquad\qquad\quad \underset{CH_3}{\vert}$ | PPG-50<br>Oleyl Ether |
| | | |
| ESTERS | $CH_3(CH_2)_7CH=CH(CH_2)_7\overset{O}{\overset{\Vert}{C}}\text{-}OCH_2\underset{\underset{CH_2OH}{\vert}}{CH}OH$ | Glyceryl Oleate |
| | | |
| AMIDES | $CH_3(CH_2)_7CH=CH(CH_2)_7\overset{O}{\overset{\Vert}{C}}\text{-}NH_2$ | Oleamide |
| | $CH_3(CH_2)_7CH=CH(CH_2)_7\overset{O}{\overset{\Vert}{C}}\text{-}N(CH_2CH_2OH)_2$ | Oleamide DEA |
| | $CH_3(CH_2)_7CH=CH(CH_2)_7\overset{O}{\overset{\Vert}{C}}\text{-}NH\text{-}CH_2CH_2OH$ | Oleamide MEA |
| | $CH_3(CH_2)_7CH=CH(CH_2)_7\overset{O}{\overset{\Vert}{C}}\text{-}NH\text{-}CH_2\underset{\underset{CH_3}{\vert}}{CH}\text{-}OH$ | Oleamide MIPA |
| | | |
| ZWITTERION | $CH_3(CH_2)_7CH=CH(CH_2)_7CH_2\text{---}\underset{\underset{CH_3}{\vert}}{\overset{\overset{CH_3}{\vert}}{N^{\pm}}}\text{---}CH_2COO$ | Oleyl Betaine |

In the preferred embodiment of the present invention, wherein the carrier is a pressure sensitive adhesive comprising a blend of polymers, an exemplary general method of preparation is as follows:

1. Appropriate amounts of soluble PVP, solvent(s), functional derivative of a fatty acid enhancer(s) , and organic solvent(s) (for example, toluene) are combined and thoroughly mixed in a vessel.
2. The drug is then added to the mixture and agitation is carried out until the drug is uniformly mixed in.
3. Appropriate amounts of polysiloxane and polyacrylate are then added to the drug mixture, and thoroughly mixed.
4. The formulation is then transferred to a coating operation where it is coated onto a protective release liner at a controlled specified thickness. The coated product is then passed through an oven in order to drive off all volatile processing solvents.

5. The dried product on the release liner is then joined to the backing material and wound into rolls for storage.

6. Appropriate size and shape "systems" are die-cut from the roll material and then pouched.

The order of steps, the amount of the ingredients, and the amount and time of agitation or mixing may be important process variables which will depend on the specific polymers, drug, cosolvents, and enhancers used in the formulation. These factors can be adjusted by those skilled in the art, while keeping in mind the object of providing a uniform product. It is believed that a number of other methods, including changing some of the order of steps, can be carried out and will give desirable results. In addition to having various shapes, the dosage units produces may come in various sizes. A surface area in the range of 1 to 200 square centimeters is contemplated, and the presently preferred sizes are: 5, 10, 15, 20, 30, 30 and 60 square centimeters.

To summarize, the preferred and optimum compositions for rubber and polyacrylate embodiments are as follows:

TABLE I

| PERCENT BY WEIGHT | | |
|---|---|---|
| Component | Preferred Range | Optimum Range |
| Rubber | 97 - 9 | 94 - 14 |
| Polyacrylate | 0 - 95 | 5 - 85 |
| PVP | 0 - 20 | 5 - 15 |
| Co-solvent(s) | 0 - 30 | 0 - 20 |
| Fatty acid derivative enhancer | 1 - 20 | 5 - 15 |
| Drug(s) | 0.05 - 50 | 0.1 - 20 |

In certain embodiments of the invention a plasticizer or tackifying agent is incorporated into the formulation to improve the adhesive characteristics of the pressure-sensitive adhesive composition.

The compositions of this invention may further be provided with various thickeners, fillers and other additives known for use with transdermal drug delivery systems. Where the composition tends to absorb water, for example, when lecithin is used as a co-solvent, hydrophilic substances are especially useful.

In a device aspect of the invention, the pressure-sensitive adhesive composition can be used as an adhesive portion of any transdermal drug delivery system (e.g., a reservoir device) or it can comprise an adhesive monolithic device. Of course, the principles of the invention would still apply to embodiments where the transdermal drug delivery composition is not a pressure-sensitive adhesive and comprises a drug reservoir.

Reference to FIG. 1 shows a schematic illustration of an adhesive monolithic device embodiment of the invention 10. The transdermal drug delivery system comprises a monolithic body 11 of a defined geometric shape with a protective release liner 12 on one side of monolithic body 11 and a backing layer 13 on the other side. Removal of the release liner 12 exposes the pressure-sensitive multiple polymer adhesive composition which functions as the drug carrier matrix, a drug penetration enhancer and as the means of applying the system to the patient.

A device, or individual dosage unit, of the present invention can be produced in any manner known to those of skill in the art. After the dermal composition is formed, it may be brought into contact with the backing layer in any manner known to those of skill in the art. Such techniques include calendar coating, hot melt coating, solution coating, etc. Of course, backing materials are well known in the art and can comprise plastic films of polyethylene, vinyl acetate resins, polyester, polypropylene, BAREX®, ethylene/vinyl acetate copolymers, polyvinyl chloride, polyurethane, and the like, metal foils, non-woven fabric, cloth, coextrusions or laminations of the above and commercially available laminates. The backing material generally has a thickness in the range of 2 to 1000 micrometers and the dermal composition is generally disposed on backing material in a thickness ranging from about 12 to 250 micrometers thick.

Suitable release liners are also well known in the art.

The configuration of the transdermal delivery system of the present invention can be in any shape or size as is necessary or desirable. Illustratively, a single dosage unit may have a surface area in the range of 1 to 200 cm$^2$. Preferred sizes are from 5 to 60 cm$^2$.

In relation to the second aspect of the invention, "enhancing penetration" refers to the acceleration of a drug through the skin or mucosa. "Topical application" means the administration of the composition of the invention to a mammal in any way which results in the physical contact of the composition with skin or mucosa at an anatomical site of interest. Those of skill in the art would know what dosages and length of use to prescribe for a given formulation, depending upon the desired therapeutic result, the type of drug and other components in the transdermal composition, the size of the transdermal composition in its final form and the age and physical state of the mammal.

In a preferred embodiment of the first aspect, the carrier is polyacrylate adhesive, the acid-labile drug is norethindrone acetate, the penetration enhancer is selected from the group consisting of oleyl alcohol and oleamide, and the solvent is a glycol.

The following specific examples are included as illustrative of pressure-sensitive adhesive compositions and transdermal drug delivery systems, and methods of making same, within the contemplation of the invention. These examples are in no way intended to be limiting of the scope of the invention.

EXAMPLE 1

| COMPONENT | PERCENT BY WEIGHT |
|---|---|
| Polysiloxane A Adhesive (BIO-PSA Q7-4503) | 5.0 |
| Polysiloxane B Adhesive (BIO-PSA X7-4603) | 71.6 |
| Polyacrylate Adhesive (GMS 737) | 5.0 |
| Oleamide | 6.0 |
| Dipropylene Glycol | 2.0 |
| Polyvinylpyrrolidone (Kollidon 30) | 5.0 |
| Polyoxyethylene (2) Oleyl Ether (Brij 93) | 2.0 |
| Norethindrone Acetate | 3.0 |
| Estradiol | 0.4 |
|  | $\overline{100.0}$ |

EXAMPLE 2

| COMPONENT | PERCENT BY WEIGHT |
|---|---|
| Polysiloxane A Adhesive (BIO-PSA Q7-4503) | 5.0 |
| Polysiloxane B Adhesive (BIO-PSA X7-4603) | 71.6 |
| Polyacrylate Adhesive (GMS 737) | 5.0 |
| Oleamide | 6.0 |
| Dipropylene Glycol | 4.0 |
| Polyvinylpyrrolidone (Kollidon 30) | 5.0 |
| Norethindrone Acetate | 3.0 |

(continued)

| COMPONENT | PERCENT BY WEIGHT |
|-----------|-------------------|
| Estradiol | 0.4 |
|           | 100.0 |

EXAMPLE 3

An estradiol/norethindrone acetate-polymer mixture is prepared by combining 0.8 parts of estradiol, 3.0 parts of norethindrone acetate, 4.0 parts of polyoxyethylene (2) oleyl ether (Brij 93), 6.0 parts of oleyl alcohol, 10.0 parts of polyvinylpyrrolidone (Kollidon VA 64), 5.0 parts of polyacrylate adhesive (GMS 737), and 71.2 parts of polysiloxane adhesive (BIO-PSA X7-4603) in an appropriate container, and mixing well until the mixture is completely homogeneous. The resulting composition has the ingredient concentrations on a "dry" basis, that is, after removal of volatile process solvents, given below.

| COMPONENT | PERCENT BY WEIGHT |
|-----------|-------------------|
| Polysiloxane adhesive (BIO-PSA X7-4603) | 71.2 |
| Polyacrylate adhesive (GMS 737) | 5.0 |
| Oleyl Alcohol | 6.0 |
| Polyoxyethylene (2) Oleyl Ether (Brij 93) | 4.0 |
| Polyvinylpyrrolidone (Kollidon 30) | 10.0 |
| Norethindrone Acetate | 3.0 |
| Estradiol | 0.8 |
|           | 100.0 |

## Claims

1. A transdermal drug penetration enhancing composition comprising (a) a therapeutically effective amount of a pharmacologically active, acid-labile drug that is subject to acid catalysed degradation; (b) a pharmaceutically acceptable carrier substantially free of a lower alkanol; and (c) a penetration-enhancing amount of a functional derivative of a fatty acid, wherein said derivative is selected from the group consisting of amides, alcohols, and polyols.

2. The composition of claim 1, wherein said functional derivative is selected from the group consisting of oleamide or oleyl alcohol.

3. The composition of claim 1 or claim 2, wherein said drug is selected from the group consisting of Androisoxazole, Bolasterone, Clostebol, Ethylestrenol, Formyldienolone, 4-Hydroxy-19-nortestosterone, Methenolone, Methyltrienolone, Nandrolone, Nandrolone Decanoate, Nandrolone *p*-Hexyloxyphenyl -propionate, Nandrolone Phenpropionate, Norbolethone, Oxymesterone, Quinbolone, Stenbolone, Trenbolone, Boldenone, Fluoxymesterone, Mestanolone, Mesterolone, Methandrostenolone, 17-Methyltestosterone, 17α-Methyl-testosterone 3-Cyclopentyl Enol Ether, Norethandrolone, Normethandrone, Oxandrolone, Oxymetholone, Prasterone, Stanlolone, Stanozolol, Testosterone, Testosterone 17-Chloral Hemiacetal, Testosterone 17β-Cypionate, Testosterone Enanthate, Testosterone Nicotinate, Testosterone Phenylacetate, Testosterone Propionate, Tiomesterone, Canrenone, Oleandrin, Spironolactone, Anagestone, Chlormadinone Acetate, Delmadinone Acetate, Demegestone, Dimethisterone, Dydrogesterone, Ethinylestrenol, Ethisterone, Ethynodiol, Ethynodiol Diacetate, Flurogestone Acetate, Gestonorone Caproate, Haloprogesterone, 17-Hydroxy-16-methylene-progesterone, 17α-Hydroxyprogesterone, 17α-Hydroxyprogesterone Caproate, Medrogestone, Medroxyprogesterone, Megestrol Acetate,Melengestrol, Norethindrone, Norethindrone Acetate, Norethynodrel, Norgesterone, Norgestimate, Norgestrel, Norgestrienone, 19-Norprogesterone, Norvinisterone, Pentagestrone, Progesterone, Promegestone, Quingestrone and Trengestone.

4. The composition of claim 1 or claim 2, wherein said drug is norethindrone acetate.

5. The composition of claim 1, 2 or 4, wherein said drug is estradiol.

6. The composition of any preceding claim, wherein said carrier is a pressure sensitive adhesive.

7. The composition of any preceding claim, which further comprises a solvent.

8. The composition of claim 7, wherein said carrier is a polyacrylate adhesive, said acid-labile drug is norethindrone acetate; said penetration enhancer is selected from the group consisting of oleyl alcohol and oleamide; and said solvent is a glycol.

9. The composition of claim 7, comprising: 3% by weight of the total composition of norethindrone acetate; 0.4% by weight of the total composition of estradiol; 5.0% by weight of the total composition of polysiloxane A adhesive; 71.6% by weight of the total composition of polysiloxane B adhesive; 5.0 % by weight of the total composition of polyacrylate adhesive; 6.0% by weight of the total composition of oleamide; 2.0% by weight of the total composition of dipropylene glycol; 5.0% by weight of the total composition of polyvinylpyrrolidone; and 2.0% by weight of the total composition of polyoxyethylene (2) oleyl ether.

10. The composition of claim 7, comprising: 3% by weight of the total composition of norethindrone acetate; 0.4% by weight of the total composition of estradiol; 5.0% by weight of the total composition of polysiloxane A adhesive; 71.6% by weight of the total composition of polysiloxane B adhesive; 5.0% by weight of the total composition of polyacrylate adhesive; 6.0% by weight of the total composition of oleamide; 4.0% by weight of the total composition of dipropylene glycol; and 5.0% by weight of the total composition of polyvinylpyrrolidone.

11. The composition of claim 7, comprising: 3% by weight of the total composition of norethindrone acetate; 0.8% by weight of the total composition of estradiol; 71.2% by weight of the total composition of polysiloxane A adhesive; 5.0% by weight of the total composition of polyacrylate adhesive; 6.0% by weight of the total composition of oleyl alcohol; 4.0% by weight of the total composition of polyoxyethylene (2) oleyl ether; and 10.0% by weight of the total composition of polyvinylpyrrolidone.

12. The use of: a) a therapeutically effective amount of a pharmacologically active, acid-labile drug which is subject to acid catalysed degradation; (b) a pharmaceutically acceptable carrier substantially free of a lower alkanol; and (c) a penetration-enhancing amount of a functional derivative of a fatty acid, wherein said derivative is selected from the group consisting of amides, alcohols, and polyols, in the manufacture of a topical medicament for enhancing the penetration of a phamaceutically active compound.

13. The use of claim 12, wherein said drug is selected from the group consisting of Androisoxazole, Bolasterone, Clostebol, Ethylestrenol, Formyldienolone, 4-Hydroxy-19-nortestosterone, Methenolone, Methyltrienolone, Nandrolone, Nandrolone Decanoate, Nandrolone $p$-Hexyloxyphenyl-propionate, Nandrolone Phenpropionate, Norbolethone, Oxymesterone, Quinbolone, Stenbolone, Trenbolone, Boldenone, Fluoxymesterone, Mestanolone, Mesterolone, Methandrostenolone, 17-Methyltestosterone, 17$\alpha$-Methyl-testosterone 3-Cyclopentyl Enol Ether, Norethandrolone, Normethandrone, Oxandrolone, Oxymetholone, Prasterone, Stanlolone, Stanozolol, Testosterone, Testosterone 17-Chloral Hemiacetal, Testosterone 17$\beta$-Cypionate, Testosterone Enanthate, Testosterone Nicotinate, Testosterone Phenylacetate, Testosterone Propionate, Tiomesterone, Canrenone, Oleandrin, Spironolactone, Anagestone, Chlormadinone Acetate, Delmadinone Acetate, Demegestone, Dimethisterone, Dydrogesterone, Ethinylestrenol, Ethisterone, Ethynodiol, Ethynodiol Diacetate, Flurogestone Acetate, Gestonorone Caproate, Haloprogesterone, 17-Hydroxy-16-methylene-progesterone, 17$\alpha$-Hydroxyprogesterone, 17$\alpha$-Hydroxyprogesterone Caproate, Medrogestone, Medroxyprogesterone, Megestrol Acetate,Melengestrol, Norethindrone, Norethindrone Acetate, Norethynodrel, Norgesterone, Norgestimate, Norgestrel, Norgestrienone, 19-Norprogesterone, Norvinisterone, Pentagestrone, Progesterone, Promegestone, Quingestrone and Trengestone.

14. A stabilised adhesive type transdermal device for delivery of a compound, said compound being stable upon extended storage of said device, comprising an effective amount of said compound in a carrier which is substantially free of lower alkanols, and a penetration-enhancing amount of a functional derivative of a fatty acid, wherein said derivative is selected from the group consisting of amides, alcohols and polyols and wherein said compound is selected from the group consisting of progesterone, ethisterone, medroxyprogesterone, 17α-hydroxyprogesterone, norethindrone, norethindrone acetate, dydrogesterone, chlomadinone acetate, norgestrel and esters.

15. The device of claim 14, wherein said carrier comprises a biocompatible polymeric adhesive.

**Patentansprüche**

1. Eine die transdermale Penetration von Arzneistoffen verbessernde Zusammensetzung, welche (a) eine therapeutisch wirksame Menge eines pharmakologisch aktiven, säurelabilen Arzneistoffs, welcher dem säurekatalysierten Abbau unterliegt; (b) eine pharmazeutisch akzeptable Trägersubstanz, welche im Wesentlichen frei von einem niederen Alkanol ist; und (c) eine penetrationsverbessernde Menge eines funktionalen Derivates einer Fettsäure, wobei das Derivat aus der Gruppe bestehend aus Amiden, Alkoholen und Polyolen ausgewählt ist, aufweist.

2. Zusammensetzung nach Anspruch 1, wobei das funktionale Derivat aus der Gruppe bestehend aus Oleamid oder Oleylalkohol ausgewählt ist.

3. Zusammensetzung nach Anspruch 1 oder 2, wobei der Arzneistoff aus der Gruppe bestehend aus Androisoxazol, Bolasteron, Clostebol, Ethylestrenol, Formyldienolon, 4-Hydroxy-19-Nortestosteron, Methenolon, Methyltrienolon, Nandrolon, Nandrolondecanoat, Nandrolon-*p*-Hexyloxyphenylpropionat, Nandrolonphenpropionat, Norbolethon, Oxymesteron, Quinbolon, Stenbolon, Trenbolon, Boldenon, Fluoxymesteron, Mestanolon, Mesterolon, Methandrostenolon, 17-Methyltestosteron, 17α-Methyltestosteron-3-Cyclopentyl-Enolether, Norethandrolon, Normethandron, Oxandrolon, Oxymetholon, Prasteron, Stanlolon, Stanozolol, Testosteron, Testosteron-17-Chloral-Hemiacetal, Testosteron-17β-Cypionat, Testosteron-Enanthat, Testosteron-Nicotinat, Testosteron-Phenylacetat, Testosteron-Propionat, Tiomesteron, Canrenon, Oleandrin, Spironolaceton, Anageston, Chlormadinonacetat, Delmadinonacetat, Demegeston, Dimethisteron, Dydrogesteron, Ethinylestrenol, Ethisteron, Ethynodiol, Ethynodioldiacetat, Flurogestonacetat, Gestonoroncaproat, Haloprogesteron, 17-Hydroxy-16-Methylenprogesteron, 17α-Hydroxyprogesteron, 17α-Hydroxyprogesteroncaproat, Medrogeston, Medroxyprogesteron, Megestrolacetat, Melengestrol, Norethindron, Norethindronacetat, Norethynodrel, Norgesteron, Norgestimat, Norgestrel, Norgestrienon, 19-Norprogesteron, Norvinisteron, Pentagestron, Progesteron, Promegeston, Quingestron und Trengeston ausgewählt ist.

4. Zusammensetzung nach Anspruch 1 oder 2, wobei der Arzneistoff Norethindronacetat ist.

5. Zusammensetzung nach Anspruch 1, 2 oder 4, wobei der Arzneistoff Estradiol ist.

6. Zusammensetzung nach einem der vorhergehenden Ansprüche, wobei die Trägersubstanz ein druckempfindlicher Klebstoff ist.

7. Zusammensetzung nach einem der vorhergehenden Ansprüche, welche ferner ein Lösemittel enthält.

8. Zusammensetzung nach Anspruch 7, wobei die Trägersubstanz ein Polyacrylatklebstoff, der säurelabile Arzneistoff Norethindronacetat, der Penetrationsverbesserer aus der Gruppe bestehend aus Oleylalkohol und Oleamid ausgewählt und das Lösemittel ein Glykol ist.

9. Zusammensetzung nach Anspruch 7, welche 3 Gew.-% der gesamten Zusammensetzung Norethindronacetat, 0,4 Gew.-% der gesamten Zusammensetzung Estradiol, 5,0 Gew.-% der gesamten Zusammensetzung Polysiloxan-A-Klebstoff, 71,6 Gew.-% der gesamten Zusammensetzung Polysiloxan-B-Klebstoff, 5,0 Gew.-% der gesamten Zusammensetzung Polyacrylatklebstoff, 6,0 Gew.-% der gesamten Zusammensetzung Oleamid, 2,0 Gew.-% der gesamten Zusammensetzung Dipropylenglykol, 5,0 Gew.-% der gesamten Zusammensetzung Polyvinylpyrrolidon und 2,0 Gew.-% der gesamten Zusammensetzung Polyoxyethylen-(2)-Oleylether aufweist.

10. Zusammensetzung nach Anspruch 7, welche 3 Gew.-% der gesamten Zusammensetzung Norethindronacetat, 0,4 Gew.-% der gesamten Zusammensetzung Estradiol, 5,0 Gew.-% der gesamten Zusammensetzung Polysiloxan-A-Klebstoff, 71,6 Gew.-% der gesamten Zusammensetzung Polysiloxan-B-Klebstoff, 5,0 Gew.-% der gesamten Zusammensetzung Polyacrylatklebstoff, 6,0 Gew.-% der gesamten Zusammensetzung Oleamid, 4,0 Gew.-% der gesamten Zusammensetzung Dipropylenglykol und 5,0 Gew.-% der gesamten Zusammensetzung Polyvinylpyrrolidon aufweist.

**11.** Zusammensetzung nach Anspruch 7, welche 3 Gew.-% der gesamten Zusammensetzung Norethindronacetat, 0,8 Gew.-% der gesamten Zusammensetzung Estradiol, 71,2 Gew.-% der gesamten Zusammensetzung Polysiloxan-A-Klebstoff, 5,0 Gew.-% der gesamten Zusammensetzung Polyacrylatklebstoff, 6,0 Gew.-% der gesamten Zusammensetzung Oleylalkohol, 4,0 Gew.-% der gesamten Zusammensetzung Polyoxyethylen-(2)-Oleylether und 10,0 Gew.-% der gesamten Zusammensetzung Polyvinylpyrrolidon aufweist.

**12.** Verwendung von (a) einer therapeutisch wirksamen Menge eines pharmakologisch aktiven, säurelabilen Arzneistoffs, welcher dem säurekatalysierten Abbau unterliegt; (b) einer pharmazeutisch akzeptablen Trägersubstanz, welche im Wesentlichen frei von einem niederen Alkanol ist; und (c) einer penetrationsverbessernden Menge eines funktionalen Derivates einer Fettsäure, wobei das Derivat aus der Gruppe bestehend aus Amiden, Alkoholen und Polyolen ausgewählt ist, bei der Herstellung eines topischen Medikamentes zur Verbesserung der Penetration einer pharmazeutisch aktiven Verbindung.

**13.** Verwendung nach Anspruch 12, wobei der Arzneistoff aus der Gruppe bestehend aus Androisoxazol, Bolasteron, Clostebol, Ethylestrenol, Formyldienolon, 4-Hydroxy-19-Nortestosteron, Methenolon, Methyltrienolon, Nandrolon, Nandrolondecanoat, Nandrolon-*p*-Hexyloxyphenylpropionat, Nandrolonphenpropionat, Norbolethon, Oxymesteron, Quinbolon, Stenbolon, Trenbolon, Boldenon, Fluoxymesteron, Mestanolon, Mesterolon, Methandrostenolon, 17-Methyltestosteron, 17$\alpha$-Methyltestosteron-3-Cyclopentyl-Enolether, Norethandrolon, Normethandron, Oxandrolon, Oxymetholon, Prasteron, Stanlolon, Stanozolol, Testosteron, Testosteron-17-Chloral-Hemiacetal, Testosteron-17β-Cypionat, Testosteron-Enanthat, Testosteron-Nicotinat, Testosteron-Phenylacetat, Testosteron-Propionat, Tiomesteron, Canrenon, Oleandrin, Spironolaceton, Anageston, Chlormadinonacetat, Delmadinonacetat, Demegeston, Dimethisteron, Dydrogesteron, Ethinylestrenol, Ethisteron, Ethynodiol, Ethynodioldiacetat, Flurogestonacetat, Gestonoroncaproat, Haloprogesteron, 17-Hydroxy-16-Methylenprogesteron, 17$\alpha$-Hydroxyprogesteron, 17$\alpha$-Hydroxyprogesteroncaproat, Medrogeston, Medroxyprogesteron, Megestrolacetat, Melengestrol, Norethindron, Norethindronacetat, Norethynodrel, Norgesteron, Norgestimat, Norgestrel, Norgestrienon, 19-Norprogesteron, Norvinisteron, Pentagestron, Progesteron, Promegeston, Quingestron und Trengeston ausgewählt ist.

**14.** Stabilisierte adhäsive transdermale Darreichungsform zur Bereitstellung einer Zusammensetzung, wobei die Zusammensetzung bei längerer Lagerung der Darreichungsform stabil bleibt, und wobei die Darreichungsform eine wirksame Menge der Zusammensetzung in einer Trägersubstanz, welche im Wesentlichen frei von niederen Alkanolen ist, sowie eine penetrationsverbessernde Menge eines funktionalen Derivates einer Fettsäure aufweist, wobei das Derivat aus der Gruppe bestehend aus Amiden, Alkoholen und Polyolen ausgewählt ist, und wobei die Zusammensetzung aus der Gruppe bestehend aus Progesteron, Ethisteron, Medroxyprogesteron, 17$\alpha$-Hydroxyprogesteron, Norethindron, Norethindronacetat, Dydrogesteron, Chlomadinonacetat, Norgestrel und Estern ausgewählt ist.

**15.** Darreichungsform nach Anspruch 14, wobei die Trägersubstanz einen biokompatiblen polymeren Klebstoff enthält.

**Revendications**

**1.** Composition favorisant la pénétration d'un médicament transdermique comprenant (a) une quantité thérapeutiquement efficace d'un médicament acide-labile pharmacologiquement actif qui est soumis à une dégradation catalysée de l'acide ; (b) un excipient pharmaceutiquement acceptable essentiellement exempt d'un alcanol inférieur ; et (c) une quantité favorisant la pénétration d'un dérivé fonctionnel d'un acide gras, ledit dérivé étant selectionné parmi le groupe se composant des amides, des alcools et des polyols.

**2.** Composition selon la revendication 1, dans laquelle ledit dérivé fonctionnel est selectionné dans le groupe se composant de l'oléamide ou de l'alcool oléylique.

**3.** Composition selon la revendication 1 ou 2, dans laquelle ledit médicament est selectionné parmi le groupe se composant d'androisoxazole, de bolastérone, de clostébol, d'éthylestrénol, de formyldiénolone, de 4-hydroxy-19-nortestostérone, de méthénolone, de méthyltriénolone, de nandrolone, de décanoate de nandrolone, de p-hexyloxyphénylpropionate de nandrolone, de phénopropionate de nandrolone, de norboléthone, d'oxymestérone, de quinbolone, de stenbolone, de trenbolone, de boldénone, de fluoxymestérone, de mestanolone, de mestérolone, de méthandrosténolone, de 17-méthyltestostérone, d'éther de 17$\alpha$-méthyltestostérone 3-cyclopentylénol, de noréthandrolone, de norméthandrone, d'oxandrolone, d'oxymétholone, de prastérone, de stanlolone, de stanozolol, de testostérone, de 17-chloralhémiacétal de testostérone, de 17β-cypionate de testostérone, d'énanthate de testostérone, de nicotinate de testostérone, de phénylacétate de testostérone, de propionate de testostérone, de tiomestérone, de canrénone, d'oléandrine, de spironolactone, d'anagestone, d'acétate de chlormadinone, d'acétate de delmadinone, de démégestone, de diméthistérone, de dydrogestérone, d'éthinylestrénol, d'éthistérone, d'éthynodiol, de diacétate d'éthynodiol, d'acétate de flurogestone, de caproate de gestonorone, d'haloprogestérone, de 17-hydroxy-16-méthylèneprogestérone, de 17α-hydroxyprogestérone, de caproate de 17α-hydroxyprogestérone, de médrogestone, de médroxyprogestérone, d'acétate de mégestrol, de mélengestrol, de noréthindrone, d'acétate de noréthindrone, de norethynodrel, de norgestérone, de norgestimate, de norgestrel, de norgestriénone, de 19-norprogestérone, de norvinistérone, de pentagestrone, de progestérone, de promégestone, de quingestrone et de trengestone.

4. Composition selon la revendication 1 ou 2, dans laquelle ledit médicament est l'acétate de noréthindrone.

5. Composition selon la revendication 1, 2 ou 4, dans laquelle ledit médicament est l'estradiol.

6. Composition selon l'une quelconque des revendications précédentes, dans laquelle ledit excipient est un adhésif sensible à la pression.

7. Composition selon l'une quelconque des revendications précédentes, qui comprend en outre un solvant.

8. Composition selon la revendication 7, dans laquelle ledit excipient est un adhésif de polyacrylate, ledit médicament acide-labile est l'acétate de noréthindrone ; ledit agent favorisant la pénétration est selectionné dans le groupe se composant de l'alcool oléylique et de l'oléamide ; et ledit solvant est un glycol.

9. Composition selon la revendication 7, comprenant : 3% en poids de la composition totale d'acétate de noréthindrone ; 0,4% en poids de la composition totale d'estradiol ; 5,0% en poids de la composition totale d'un adhésif de polysiloxane A ; 71,6% en poids de la composition totale d'un adhésif de polysiloxane B ; 5,0% en poids de la composition totale d'un adhésif de polyacrylate ; 6,0% en poids de la composition totale d'oléamide ; 2,0% en poids de la composition totale du dipropylène glycol ; 5,0% en poids de la composition totale de polyvinylpyrrolidone ; et 2,0% en poids de la composition totale d'éther de polyoxyéthylène oléylique (2).

10. Composition selon la revendication 7, comprenant : 3% en poids de la composition totale d'acétate de noréthindrone ; 0,4% en poids de la composition totale d'estradiol ; 5,0% en poids de la composition totale d'un adhésif de polysiloxane A ; 71,6% en poids de la composition totale d'un adhésif de polysiloxane B ; 5,0% en poids de la composition totale d'un adhésif de polyacrylate ; 6,0% en poids de la composition totale d'oléamide ; 4,0% en poids de la composition totale de dipropylène glycol ; et 5,0% en poids de la composition totale de polyvinylpyrrolidone.

11. Composition selon la revendication 7, comprenant : 3% en poids de la composition totale d'acétate de noréthindrone ; 0,8% en poids de la composition totale d'estradiol ; 71,2% en poids de la composition totale d'un adhésif de polysiloxane A ; 5,0% en poids de la composition totale d'un adhésif de polyacrylate ; 6,0% en poids de la composition totale d'alcool oléylique ; 4,0% en poids de la composition totale d'éther de polyoxyéthylène oléylique (2) ; et 10,0% en poids de la composition totale de polyvinylpyrrolidone.

12. Utilisation de : (a) une quantité thérapeutiquement efficace d'un médicament acide-labile pharmacologiquement actif qui est soumis à une dégradation catalysée de l'acide ; (b) un excipient pharmaceutiquement acceptable essentiellement exempt d'un alcanol inférieur ; et (c) une quantité favorisant la pénétration d'un dérivé fonctionnel d'un acide gras, dans lequel ledit dérivé est selectionné dans le groupe se composant des amides, des alcools et des polyols, dans la fabrication d'un médicament topique destiné à favoriser la pénétration d'un composé pharmaceutiquement actif.

13. Utilisation selon la revendication 12, dans laquelle le médicament est selectionné dans le groupe se composant d'androisoxazole, de bolastérone, de clostébol, d'éthylestrénol, de formyldiénolone, de 4-hydroxy-19-nortestostérone, de méthénolone, de méthyltriénolone, de nandrolone, de décanoate de nandrolone, de p-hexyloxyphénylpropionate de nandrolone, de phénopropionate de nandrolone, de norboléthone, d'oxymestérone, de quinbolone, de stenbolone, de trenbolone, de boldénone, de fluoxymestérone, de mestanolone, de mestérolone, de méthandrosténolone, de 17-méthyltestostérone, d'éther de 17α-méthyltestostérone 3-cyclopentylénol, de noréthandrolone, de norméthandrone, d'oxandrolone, d'oxymétholone, de prastérone, de stanlolone, de stanozolol, de testostérone, de 17-chloral hémiacétal de testostérone, de 17β-cypionate de testostérone, d'énanthate de testostérone, de nicotinate de testostérone, de phénylacétate de testostérone, de propionate de testostérone, de tiomestérone, de canrénone, d'oléandrine, de spironolactone, d'anagestone, d'acétate de chlormadinone, d'acétate de delmadinone, de démégestone, de diméthistérone, de dydrogestérone, d'éthinylestrénol, d'éthistérone, d'éthynodiol, de diacétate d'éthynodiol, d'acétate de fluorogestone, de caproate de gestonorone, d'haloprogestérone, de 17-hydroxy-16-méthylèneprogestérone, de $17\alpha$-hydroxyprogestérone, de caproate de $17\alpha$-hydroxyprogestérone, de médrogestone, de médroxyprogestérone, d'acétate de mégestrol, de mélengestrol, de noréthindrone, d'acétate de noréthindrone, de norethynodrel, de norgestérone, de norgestimate, de norgestrel, de norgestriénone, de 19-norprogestérone, de norvinistérone, de pentagestrone, de progestérone, de promégestone, de quingestrone et de trengestone.

**14.** Dispositif transdermique de type adhésif stabilisé pour l'administration d'un composé, ledit composé étant stable lors d'un stockage prolongé dudit dispositif, comprenant une quantité efficace dudit composé dans un excipient qui est essentiellement exempt d'alcanol inférieur, et d'une quantité favorisant la pénétration d'un dérivé fonctionnel d'un acide gras, dans lequel ledit dérivé est selectionné dans le groupe se composant des amides, des alcools et des polyols, et dans lequel ledit composé est selectionné dans le groupe se composant de progestérone, d'éthistérone, de médroxyprogestérone, de $17\alpha$-hydroxyprogestérone, de noréthindrone, d'acétate de noréthindrone, de dydrogestérone, d'acétate de chlomadinone, de norgestrel et d'esters.

**15.** Dispositif selon la revendication 14, dans lequel ledit excipient comprend un adhésif polymère biocompatible.

# FIG. 1